(19) Europäisches Patentamt

European Patent Office

Office européen des brevets

(11) **EP 1 398 630 B1**

(12) **FASCICULE DE BREVET EUROPEEN**

(45) Date de publication et mention
de la délivrance du brevet:
**22.03.2006 Bulletin 2006/12**

(51) Int Cl.:
**G01N 33/24** (2006.01)        **G01V 3/32** (2006.01)
**E21B 49/00** (2006.01)

(21) Numéro de dépôt: **03291965.6**

(22) Date de dépôt: **06.08.2003**

(54) **Méthode pour déterminer l'indice de résistivité en fonction de la saturation en eau, de certaines roches de porosité complexe**

Verfahren zur Bestimmung des Widerstandsindex, als Funktion der Wassersättigung, von bestimmten Gesteinsproben mit komplexer Porosität

Method for determining the resistivity index, as a function of the water saturation, of certain rocks with complex porosity

(84) Etats contractants désignés:
**BE DE GB IT NL**

(30) Priorité: **11.09.2002 FR 0211282**

(43) Date de publication de la demande:
**17.03.2004 Bulletin 2004/12**

(73) Titulaire: **Institut Français du Pétrole
92852 Rueil-Malmaison Cedex (FR)**

(72) Inventeur: **Fleury, Marc
78170 La Celle-Saint-Cloud (FR)**

(56) Documents cités:
**EP-A- 0 974 839**

- **SEN P N: "Resistivity of partially saturated carbonate rocks with microporosity" GEOPHYSICS, MARCH-APRIL 1997, SOC. EXPLORATION GEOPHYSICISTS, USA, vol. 62, no. 2, pages 415-425, XP002245236 ISSN: 0016-8033**

- **OHEN H A ET AL: "NMR Relaxivity Grouping or NMR Facies Identification is Key to Effective Integration of Core Nuclear Magnetic Resonance Data with Wireline Log" SCA PROCEEDINGS, PAPER 9942, 1999, XP002245237**
- **MIROTCHNIK K ET AL: "Determination of Mud Invasion Characteristics of Sandstone Reservoirs Using a Combination of Advanced Core Analysis Techniques" SCA PROCEEDINGS, PAPER 9815, 1998, XP002245238**
- **SWANSON B E: "MICROPOROSITY IN RESERVOIR ROCKS: ITS MEASUREMENT AND INFLUENCE ON ELECTRICAL RESISTIVITY" LOG ANAL NOV-DEC 1985, vol. 26, no. 6, novembre 1985 (1985-11), pages 42-52, XP008018722**
- **FLEURY M: "CARACTERISATION DE STRUCTURES POREUSES PAR RELAXOMETRIE RMN" REVUE DE L'INSTITUT FRANCAIS DU PETROLE, EDITIONS TECHNIP. PARIS, FR, vol. 53, no. 4, juillet 1998 (1998-07), pages 489-493, XP000831361 ISSN: 1294-4475**
- **ARCHIE G E: "THE ELECTRICAL RESISTIVITY LOG AS AN AID IN DETERMINING SOME RESERVOIR CHARACTERISTICS" PETROLEUM DEVELOPMENT AND TECHNOLOGY, XX, XX, vol. 146, 1942, pages 54-62, XP008001923**

EP 1 398 630 B1

## Description

La présente invention concerne une méthode pour déterminer l'indice de résistivité RI en fonction de la saturation en eau, de certaines roches de porosité complexe.

La méthode selon l'invention trouve des applications notamment dans le domaine de l'exploitation de gisements d'hydrocarbures.

L'évaluation des réservoirs carbonatés est une tâche particulièrement difficile pour les pétrophysiciens, et l'on manque toujours de connaissances précises concernant les propriétés de transport au sein de ces milieux poreux. Par rapport aux roches siliclastiques, les carbonates peuvent être plus simples sur le plan minéralogique, mais ils sont incomparablement plus complexes en termes de structure poreuse et de propriétés de surface. L'origine largement biologique des sédiments, alliée à différents processus diagénétiques, donne lieu à des structures poreuses complexes qui peuvent être extrêmement différentes d'un réservoir à l'autre. Pour de nombreux systèmes carbonatés, les étalonnages-de données de résistivité réalisés en laboratoire sont en contradiction avec les observations faites sur le terrain (production anhydre, pression capillaire) et avec les mesures directes de saturation en eau effectuées sur des carottes préservées.

## Etat de la technique

L'état de la technique est défini notamment par les publication suivantes :

- Bouvier L. et al, Reconciliation of Log and Laboratory Derived Irreductible Water Saturation in a Double Porosity reservoir, Advances in Core Evaluation, edited by Worthington and Longeron, Gordon and Breach Science Publishers ;

- Dixon J.R. et al (1990), The Effect of Bimodal pore Size Distribution on Electrical Properties of some Middle Eastern Limestone, Soc. Petr. Eng. 20601, 7th SPE Middle Eastern Oil Show, Bahrain, pp.743-750 ;

- Fleury M. (1998), "FRIM : a Fast Resistivity Index Measurement Method", Proceedings of the International Symposium of the SZociety of the Core Analysts, Den Hague;

- Fleury M. et al (2000) "Frequecncy Effect on Resistivity Index Curves Using a New Method", Proceedings of the 41st Annual SPWLA Symposium, Dallas ;

- Moore C.H., (2001) "Carbonate reservoirs, Porosity Evaluation and Diagenesis in a Séquence Stratigraphic Framework", Developments in Sedimentology 55, Elsevier Editions ;

- Petricola M.J.C. et al, (1995), "Effect of Microporosity in Carbonates: Introduction of a Versatile Saturation Equation", Soc. Petr. Eng. 29841, SPE Middle Eastern Oil Show, Bahrain, pp.607-615 ;

- Sen P.N. et al (1997), "Resistivity of Partially Saturated Rocks with Microporosity", Geophysics, Vol.62, N°2, pp. 415-425.

La compréhension et la prédiction de l'effet de la structure du réseau poreux de la mouillabilité et des propriétés électriques de roches carbonatées, constituent un véritable défi scientifique, tant sur le plan théorique qu'expérimental. La correcte évaluation de ces paramètres a en effet une incidence majeure sur l'estimation du pétrole en place, notamment pour les gisements pétroliers géants du Moyen-Orient, car l'écart par rapport aux valeurs standard des exposants d'Archie m et n par rapport à la valeur 2 est si important que l'estimation de la saturation en eau peut varier de plus de 20 %.

Différentes observations expérimentales ont montré l'existence de populations de pores distinctes: micropores, macropores et mésopores, présentant des degrés de coexistence divers. En général, la courbe d'indice de résistivité $RI(Sw)=Rt(Sw)/Ro$, où Rt est la résistivité de la roche à une saturation en eau Sw, et Ro la résistivité pour Sw=1, ne peut pas être décrite par une loi de puissance (seconde loi d'Archie $RI=Sw^{-n}$), cad n est fonction de la saturation elle-même. La microporosité peut agir comme un chemin parallèle pour le courant, ce qui entraîne une diminution des valeurs de n et, de ce fait, une insensibilité graduelle de la résistivité à la saturation, comme cela a été observé sur des sables argileux. La microporosité peut être aussi la cause des faibles valeurs de n mesurées (typiquement 1,45). On a aussi observé que n peut augmenter considérablement dans certaines conditions, et qu'il existerait un lien entre la courbe RI (Sw) et la courbe de pression capillaire. L'augmentation des valeurs de n est également un effet connu de la mouillabilité, qui tend à favoriser la discontinuité de la phase aqueuse et ainsi à augmenter la résistivité par rapport à la mouillabilité à l'eau. L'effet de la mouillabilité peut entraîner soit une augmentation soudaine de n, soit une valeur n élevée sans discontinuité. Une confusion est de ce fait possible.

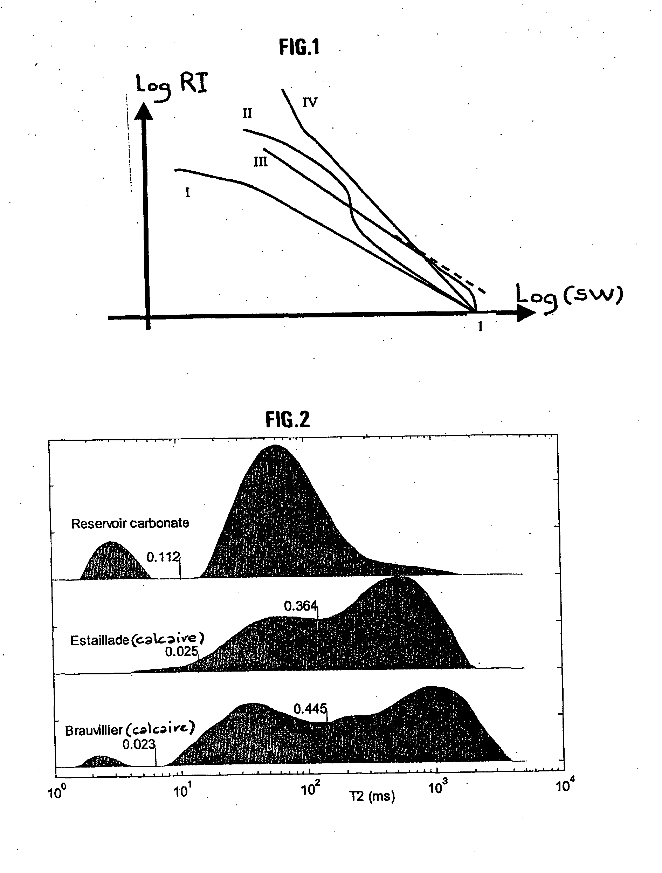

Les courbes expérimentales RI(Sw) qui ont été établies à la suite de divers travaux antérieurs déjà cités et de nos propres observations, peuvent présenter (Fig.1) quatre formes distinctes qui ne satisfont pas toujours aux lois d'Archie :

- type 1 : peut être typique de carbonates provenant de la formation Thamama,

- type II : se redressant à une saturation intermédiaire et s'aplatissant à faible saturation (la présente étude),

- type III : pente unique à faible saturation, extrapolation à Sw=1 au-dessus de Ir=1, et

- type IV : typique de systèmes mouillables à l'huile, valeurs élevées de n pouvant augmenter encore à faible saturation. Ceci est également valable pour les clastes.

On voit donc que lorsqu'on se trouve en présence de roches à la porosité complexe qui ne satisfont pas aux lois d'Archie, un grand nombre de mesures coûteuses est nécessaire pour tenir compte de la variabilité de la structure poreuse.

**La méthode selon l'invention**

La méthode selon l'invention a pour objet de déterminer les variations de l'indice de résistivité (RI) d'une famille d'échantillons de roche à structure poreuse complexe en fonction de la saturation en eau (Sw), en présence d'un fluide non conducteur. Elle comporte les étapes suivantes :

- pour chaque échantillon de la famille comprenant au moins un premier et un deuxième réseaux de pores, on détermine la fraction volumique occupée par chaque réseau de pores, par application aux différents échantillons d'une technique de relaxométrie de type RMN ;

- pour chaque échantillon de la famille, on mesure par injection de mercure la distribution des seuils de pores dans les différents réseaux de pores pour déduire les valeurs des saturations moyennes;

- on détermine expérimentalement sur un échantillon au moins de la famille servant de référence, les valeurs de coefficients reliant la variation de sa résistivité électrique en fonction de sa saturation en eau ; et

- on détermine l'indice de résistivité (RI) de tous les échantillons de la famille en se basant sur la variation de paramètres décrivant l'agencement du réseau poreux et en utilisant les valeurs des coefficients mesurés sur l'échantillon de référence.

Suivant un premier mode de mise en oeuvre convenant pour les échantillons comportant deux réseaux de pores, on détermine à partir de l'échantillon de référence, les valeurs des coefficients reliant la conductivité totale de l'échantillon aux conductivité des premier et deuxième réseaux de pores et aux saturations respectives en eau des deux réseaux, l'indice de résistivité étant calculé à partir des fractions volumiques respectives des deux réseaux de pores et de la valeur de la saturation moyenne à partir de laquelle le réseau ayant les plus petits pores, est envahi par le fluide non conducteur.

Suivant un deuxième mode de mise en oeuvre convenant pour les échantillons comportant un troisième réseau de pores, on détermine à partir de l'échantillon de référence, les valeurs des coefficients reliant la conductivité totale de l'échantillon aux conductivité des premier et deuxième réseaux de pores et aux saturations respectives en eau des deux premiers réseaux, l'indice de résistivité étant calculé à partir des fractions volumiques respectives des trois réseaux de pores, de la valeur de la saturation moyenne à partir de laquelle le réseau parmi les deux premiers réseaux de pores qui a les plus petits pores est envahi par le fluide non conducteur, et de la valeur de la saturation moyenne à partir de laquelle le réseau ayant les plus grands pores parmi les deux premiers réseaux de pores, est envahi par le fluide non conducteur.

Les variations de l'indice de résistivité obtenues par application de la méthode, s'ajustent au mieux aux courbes expérimentales dont les exemples sont illustrés à la Fig.1. La méthode s'appuie sur une technique expérimentale puissante permettant de mesurer une courbe d'indice de résistivité continue au lieu de la technique classique qui décrit la courbe RI seulement par un nombre limité de points à l'équilibre capillaire.

**Présentation succincte des figures**

Les caractéristiques et avantages de la méthode et du dispositif selon l'invention, apparaîtront plus clairement à la lecture de la description ci-après en se référant aux dessins annexés où :

- la figure 1 montre une représentation schématique en coordonnées logarithmiques de différents types de courbes RI en fonction de la saturation en eau Sw observées expérimentalement; les courbes I à III sont dus à la structure poreuse, la courbe IV est typique des effets de la mouillabilité et n'est pas spécifique aux carbonates ;

- la figure 2 montre une distribution des tailles de pores de trois carbonates étudiés : (carbonate de gisement, calcaire d'Estaillade et calcaire de Brauvillier) obtenue par RMN ; les chiffres indiquent la fraction volumique de pore à gauche du segment vertical ;

- les figures 3a, 3b montrent la sensibilité du modèle à deux réseaux de pores (DPC) à la saturation Sc à laquelle les micropores sont envahis par l'huile (3a) et à $\alpha$, rapport de conductivité initiale des deux populations de pores (3b) ;

- les figures 4a, 4b montrent l'application du modèle DPC à l'échantillon RC à la température ambiante (4a) et dans les conditions de gisement avec de l'huile morte (4b); la courbe expérimentale est en traits fins et le modèle en trait gras; les paramètres $n_1$, $n_2$ et $\alpha$ sont ajustés, $f_1$ et Sc sont mesurés ;

- la figure 5 montre un scénario d'invasion dans le modèle à trois réseaux de pores (TPC) ;

- la figure 6 montre un agencement électrique modélisant les trois réseaux dans le modèle TPC ;

- la figure 7 montre une courbe RI mesurée (trait fin) et modèle (trait gras) pour l'échantillon EL (calcaire d'Estaillade) ;

- la figure 8 montre une courbe RI mesurée (trait fin) et modèle (trait gras) pour l'échantillon BL (calcaire de Brauvillier) ; et

- la figure 9 montre une séquence de calibrage simplifié pour les diagraphies de résistivité ; pour les roches de type non-Archie, Sw(RI) est nécessaire ; les données de laboratoire fournissent généralement la relation RI(Sw).

**Description détaillée**

On dispose d'un certain nombre d'échantillons d'une même famille et l'on veut calculer l'indice de résistivité de chacun d'eux. La méthode que l'on va décrire permet d'obtenir ces indices, essentiellement à partir :

- de mesures expérimentales effectuées sur chaque échantillon de la famille, donnant le nombre de réseaux de pores de tailles différentes et la fraction volumique occupée par chaque réseau de pores ainsi que la distribution des seuils de pores pour chacun d'eux ; et

- d'une mesure expérimentale faite sur un seul échantillon de la famille, servant de référence, de la variation de sa résistivité électrique en fonction de sa saturation en eau (Sw).

Nomenclature

Cto = conductivité totale à Sw= 1

$C_i$ = conductivité de la i-ième population à $Sw_i$=1

$f_i$ = fraction volumique de pores RMN de la i-ième population, i=1,2,3

RI = indice de résistivité, Rt/RoSw = saturation moyenne en eau

$Sw_i$ = saturation en eau de la i-ième population de pores

Sc = saturation moyenne à laquelle les micropores sont envahis

Sm = saturation moyenne à laquelle les macropores sont envahis

$\alpha_{1,2}$ = rapport de conductivité $C_1/C_2$ et $C_1/C_3$ respectivement.

**Données expérimentales sur échantillons**

*Mesures de temps de relaxation RMN*

Dans un premier temps, on détermine par une méthode de relaxométrie de type RMN bien connue des spécialistes, les différents réseaux de pores à l'intérieur des échantillons de roche et la fraction volumétrique occupée par chaque réseau. Un exemple d'une telle méthode de relaxométrie d RMN est décrit par exemple dans la demande de brevet français EN 02/... du demandeur.

Nous présentons les mesures réalisées sur trois échantillons de carbonate sélectionnés. En leur appliquant une méthode de relaxométrie de type RMN bien connue des spécialistes, on peut voir que tous les échantillons se caractérisent par une distribution des tailles de pores double ou triple. Le premier est un échantillon de carbonate de gisement provenant du Moyen-Orient classé parmi les "packstones". Il a été nettoyé au moyen de différents solvants à température élevée avant de procéder aux mesures. A conditions ambiantes, l'expérience a été effectuée avec de l'huile raffinée et de la saumure de réservoir synthétique. L'expérience réalisée dans des conditions de gisement a été conduite avec de l'huile de gisement dégazée filtrée à la pression de réservoir. Après nettoyage, la mouillabilité de l'échantillon se caractérise par une mouillabilité modérée à l'eau et, après vieillissement, par une forte mouillabilité à l'huile. Les deux autres échantillons sont des carbonates d'affleurement (mouillables à l'eau). La porosité du calcaire de Brauvillier (BL) est essentiellement intergranulaire et due au cortex de l'oolite. La porosité du calcaire d'Estaillade est à la fois inter et intragranulaire. Les populations de pores sont séparées par un facteur 10 au moins.

*Mesures d'indice de résistivité*

On mesure ensuite l'indice de résistivité des échantillons. On peut utiliser avantageusement la méthode de mesure rapide de l'indice de résistivité (dite FRIM) telle que décrite dans le brevet EP 974 839 du demandeur.

Suivant cette méthode, on réalise un déplacement forcé huile-eau sur une petite carotte de 2,5 cm de long et 4 cm de diamètre par exemple. Ce déplacement est très proche d'un procédé de déplacement à paroi semiperméable, excepté que l'équilibre capillaire n'est pas nécessaire. A conditions ambiantes, seuls deux ou trois paliers de pression sont utilisés. Aux conditions de gisement, lors de l'utilisation d'huile morte ou brute, on fait durer chaque palier de pression le temps nécessaire pour obtenir la stabilisation de la mouillabilité à une saturation donnée. Le drainage s'effectue ainsi en approximativement deux à trois semaines, ce qui correspond de manière typique à la cinétique des processus chimiques liés au vieillissement. La simplicité de cette méthode tient au fait qu'il suffit d'enregistrer et de reporter en temps réel la saturation et la résistance moyennes pour obtenir une courbe d'indice de résistivité continue exempte de tout artefact. Le point-clé réside dans le fait que la géométrie radiale des électrodes permet d'examiner la totalité du volume de l'échantillon et de compenser le profil de saturation non-uniforme qui apparaît en l'absence d'équilibre capillaire.

A conditions ambiantes, nous avons utilisés la cellule à électrodes radiales décrite par exemple dans le brevet EP-A-974 839 déjà cité. Les mesures d'impédance complexe ont été effectuées à une fréquence fixe de 1 kHz et la partie réelle a été extraite pour calculer l'indice de résistivité. La pression capillaire imposée la plus élevée était de 12 bars (pour une tension interfaciale $\gamma$=35 mN/m ; aux conditions de gisement, avec de l'huile brute, la pression capillaire maximale a été réduite proportionnellement à la tension interfaciale).

**Modèles de conductivité**

*Modèle de conductivité de porosité double (DPC)*

L'objectif est d'expliquer le fléchissement des courbes RI à faible saturation (type I, figure 1). Le modèle de double porosité considéré ici est pour l'essentiel très proche de ceux proposés pour les sables argileux, où les argiles présentes à la surface des pores constituent un chemin parallèle pour le courant. Pour les carbonates, nous admettons l'existence de deux réseaux de pores présentant des conductivités électriques parallèles. Les deux principaux ingrédients dans notre modèle sont la description de l'invasion des réseaux de pores en cours de drainage, et la description de l'agencement électrique des deux différentes populations de pores.

Le premier réseau 1 (macropores par exemple) représente la majeure partie du volume de pores, tandis que le deuxième réseau 2 (micropores) n'en représente qu'une petite fraction, qui n'est pas nécessairement supérieure aux seuils de percolation. Dans un premier temps, nous considérons la saturation de chaque réseau, $Sw_1$ et $Sw_2$, qui sont

reliées à la saturation moyenne mesurée Sw selon :

$$Sw = f_1 Sw_1 + f_2\, Sw_2 \;\; \text{où} \;\; f_1 + f_2 = 1 \quad (1).$$

$f_1$ et $f_2$ représentent la fraction volumique de pore de chaque population. Ces fractions ont été évaluées au moyen de la relaxométrie RMN. Dans un deuxième temps, on admet que les réseaux sont envahis par de l'huile à différentes pressions capillaires ; l'huile accède aux pores de petite taille à une pression supérieure à celle observée pour les pores de grande taille. A partir de la courbe de pression capillaire établie précédemment, cette pression correspond à une saturation moyenne Sc qui peut être déduite des courbes obtenues par injection de mercure (indiquant la distribution des seuils de pores). $Sw_1$ peut être exprimé en fonction de Sw à saturation Sw élevée :

$$Sw_1 = \frac{Sw + f_1 - 1}{f_1}, \; Sw_2 = 1 \quad pour \quad Sw \geq Sc \qquad (2)$$

En dessous de Sc, les relations $Sw_1 = f(Sw)$ et $Sw_2 = f(Sw)$ nécessitent d'autres hypothèses. On admettra (i) une relation linéaire et (ii) que $Sw_1 \rightarrow 0$, $Sw_2 \rightarrow 0$ quand $Sw \rightarrow 0$.
On en déduit que :

$$Sw_1 = \frac{f_1 + Sc - 1}{f_1 Sc} Sw,$$

$$Sw_2 = \frac{Sw}{Sc} \quad pour \quad Sw \leq Sc \qquad (3)$$

Nous considérons maintenant la conductivité de chaque réseau. Dans le cas d'une saturation initiale en saumure, la conductivité totale $Ct_0$ pour les deux réseaux en parallèle sera :

$$Ct_0 = C_1 + C_2 = C_1 (1 + \alpha) \;\; \text{où} \;\; C_2 = \alpha\, C_1 \quad (4).$$

Le paramètre $\alpha$ est le rapport de conductivité des deux réseaux saturés à 100 %. A partir de la première loi d'Archie, on suppose que $C_1$ et $C_2$ sont reliés à la fraction volumique de pore de chaque population et que, de ce fait, $\alpha$ est de l'ordre du rapport $f_2/f_1$. Lorsque les deux réseaux sont envahis par l'huile, on admet que chaque conductivité est liée à la saturation par une loi de puissance (comme dans la seconde loi d'Archie). La conductivité totale dans les deux domaines de saturation est la suivante :

$$Ct = Sw_1^{n1}\, C_1 + C_2 \quad pour\; Sw \geq Sc \qquad (5)$$

$$Ct = Sw_1^{n1}\, C_1 + Sw_2^{n2}\, C_2 \quad pour\; Sw \leq Sc \qquad (6).$$

Au moyen des équations 4, 5 et 6, on trouve l'indice de résistivité RI suivant :

$$RI = \frac{Ct_o}{Ct} = Sw_1^{-n1} \frac{1+\alpha}{1+\alpha\ Sw_1^{-n1}} \ pour\ Sw \geq Sc \qquad (7)$$

$$RI = Sw_1^{-n1} \frac{1+\alpha}{1+\alpha\ Sw_1^{-n1}/Sw_2^{-n2}} \ pour\ Sw \leq Sc \qquad (8)$$

Lorsqu'une population de pores domine (le réseau 1 dans le cas présent), les fonctions RI sont régies par les propriétés de résistivité ($n_1$) de cette population. Les équations 7 et 8 sont essentiellement similaires aux formules utilisées pour les sables argileux, excepté que nous introduisons un second exposant $n_2$ qui caractérise le second réseau.

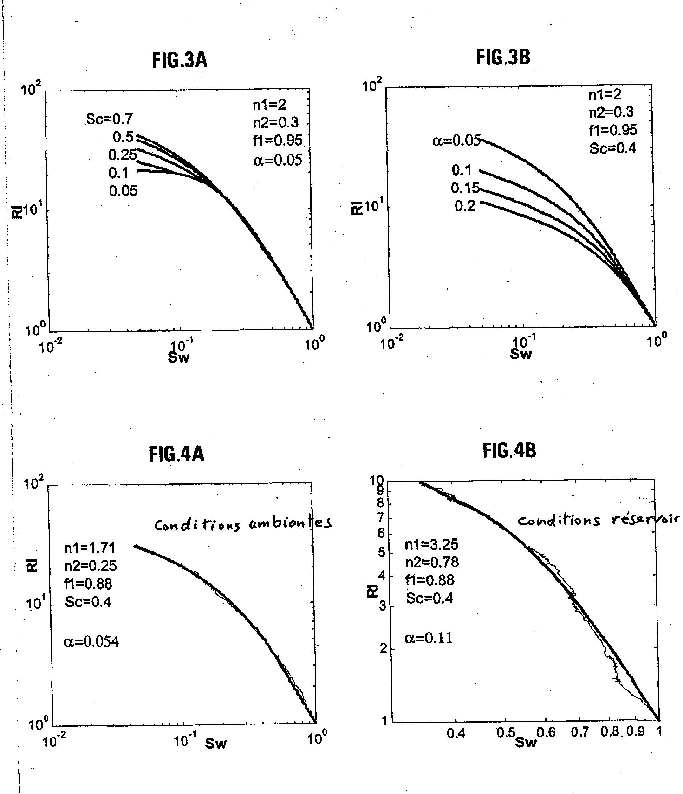

Dans le modèle DPC, il y a au total 4 paramètres $n_1$, $n_2$, $\alpha$ et Sc. Pour une courbe expérimentale donnée, Sc est mesuré séparément lors d'une expérience d'injection de mercure, alors que les autres paramètres sont ajustés. On observe toutefois un intervalle de variation de $\alpha$ autour de $f_2/f_1$ pour lequel il existe une explication physique. A saturation Sw élevée, la pente à l'échelle bilogarithmique de RI(Sw) est - $n_1$, et à faible saturation, la pente est - $n_2$. Dans une certaine mesure, Sc et $\alpha$ se compensent l'un l'autre (figure 3), mais $\alpha$ est le paramètre le plus sensible qui contrôle la valeur finale de RI. On notera que le cas Sc=0,05 présenté sur la figure 3 correspond à une situation où le deuxième réseau n'est pas envahi par l'huile, ce qui donne une asymptote horizontale.

Les paramètres du modèle ont été ajustés en vue de correspondre à la courbe expérimentale RI mesurée sur l'échantillon RC. La valeur de Sc=0,4 a été déduite du coude de la courbe de pression capillaire obtenue par injection de mercure et la fraction volumique $f_1$=0,88 de macropores (réseau 1) a été déduite par RMN (figure 2). Malgré la faible perméabilité de l'échantillon, la saturation la plus faible obtenue est très basse (4 %), ce qui permet une bonne détermination des paramètres du modèle aux conditions ambiantes. La pente initiale de la courbe RI est $n_1$=1,71 ; elle caractérise le réseau 1. Le réseau 2 est très peu sensible à la saturation ($n_2$=0,25) et ne se comporte pas comme un réseau standard. Toutefois, le rapport de conductivité initiale $\alpha$=0,054 du réseau 1 à 2 est de l'ordre de grandeur de $f_2/f_1$=0,136. Aux conditions de gisement (conditions de mouillabilité à l'huile), on observe une forte augmentation de $n_1$, mais la courbe RI n'est toujours pas linéaire à l'échelle bilogarithmique. Les caractéristiques $n_2$=0,78 et $\alpha$=0,11 du deuxième réseau sont aussi légèrement modifiées, mais la précision sur ces paramètres est plus faible qu'avec les conditions ambiantes car la saturation atteinte (à la même pression capillaire) est très supérieure et proche de Sc.

*Formule approchée dans le cas du modèle DPC*

Nous proposons une formule approchée réduisant les relations 7 et 8 à une formule unique, qui est valide pour les courbes de type I. Nous notons d'abord que :

$$Sw_2^{n2}\ Sw_1^{-n1} \approx Sc^{-n2}\ Sw^{n2-n1} \approx Sw^{n2-n1}$$

$$avec\ Sw_1 \cong Sw\ et\ Sc^{-n2} \approx 1 \qquad (15)$$

ce qui donne $f_2 \ll f_1$. Comme $n_2 \ll n_1$, les équations 7 et 8 peuvent être approchées par :

$$RI = Sw^{-n1} \frac{1+C}{1+CSw^{n2-n1}} \qquad (16).$$

Comme le montre la figure 10, l'utilisation des 3 paramètres $n_1$, $n_2$ et C suffit pour décrire avec précision les données au moyen de l'équation 16. Ici, la signification de C est approximativement la même que $\alpha$ et peut dépendre de la température. La formulation donnée dans l'équation 16 peut être utilisée pour décrire des expériences standard réalisées à l'équilibre capillaire pour lesquelles on ne dispose que d'un nombre limité de points.

*Modèle de conductivité de porosité triple (TPC)*

L'objectif est d'expliquer le redressement des courbes RI à saturation intermédiaire ou élevée, et leur inflexion à faible saturation à l'échelle bilogarithmique (types II et III, figure 1). Nous nous servons ici de l'idée générale selon laquelle les carbonates complexes peuvent avoir trois populations de pores appelées, dans un souci de simplification, micro, macro et mésopores (3, 2 et 1 respectivement). Comme dans le modèle de porosité double, on considère la saturation des 3 populations :

$$Sw = f_1 Sw_1 + f_2 Sw_2 + f_3 Sw_3 \quad \text{où} \quad f_1 + f_2 + f_3 = 1 \qquad (9)$$

Là encore, on admet que l'invasion de ces populations par l'huile au cours du drainage est séquentielle. Si le réseau 1 est envahi le premier, on définit une saturation moyenne Sm à laquelle le réseau 2 est envahi :

$$Sw_1 = \frac{Sw - f_2 - f_3}{f_1},$$

$$Sw_2 = 1 \quad Sw_3 = 1 \quad pour \quad Sw \geq Sm \qquad (10)$$

En dessous de Sm, il est possible d'imaginer de nombreux scénarios. En général, on admet des relations linéaires pour les fonctions $Sw_1(Sw)$ et $Sw_2(Sw)$. Selon un scénario possible, on admettra que $Sw_1 \to 0$, $Sw_2 \to 0$ lorsque $Sw \to Sc$. Sc est la saturation à laquelle la microporosité est envahie. Il s'ensuit que :

$$Sw_1 = \frac{Sw - f_2 Sw_2 - f_3}{f_1}, \; Sw_2 = \frac{Sw - Sc}{Sm - Sc},$$

$$Sw_3 = 1 \quad pour \quad Sc \leq Sw \leq Sm \qquad (11)$$

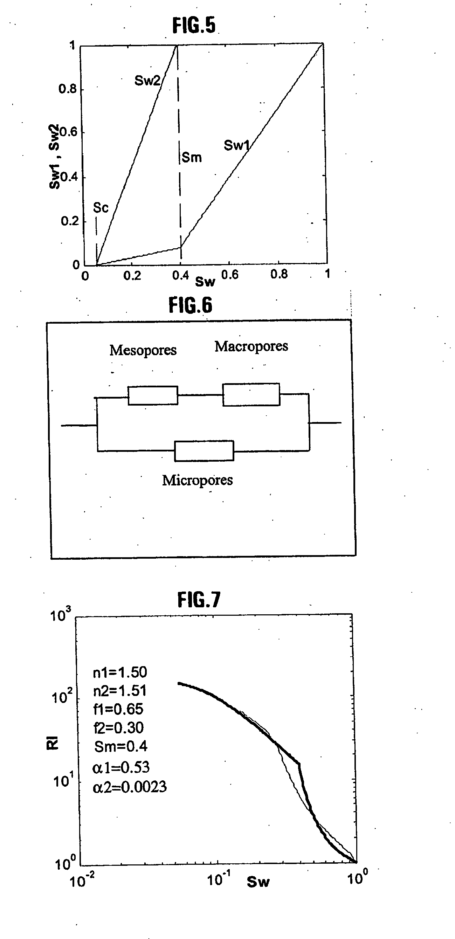

Ce scénario d'invasion est résumé sur la figure 5. Typiquement, on pense à une situation où $f_3 \ll f_1$ et $f_1 \approx f_2$, et la microporosité (réseau 3) est envahie à une pression beaucoup trop élevée pour pouvoir être observée au cours de l'expérience.

Considérons maintenant la conductivité de ces populations. On admet que les réseaux 1 et 2 sont en série, tandis que le réseau 3 est en parallèle comme le montre la figure 6. La disposition en série peut sembler en contradiction avec une invasion séquentielle par l'huile (qui est un mécanisme essentiellement parallèle), mais une telle situation est possible. A Sw=1, la conductivité $Ct_0$ du système représenté sur la figure 6 est la suivante :

$$Ct_0 = (C_1^{-1} + C_2^{-1})^{-1} + C_3 = C_1 [(1 + \alpha_1^{-1})^{-1} + \alpha_2]$$

$$\text{où } C_2 = \alpha_1 C_1, \; C_3 = \alpha_2 C_1 \qquad (12).$$

Dans les deux domaines de saturation, on obtient :

$$Ct = C_1 [(Sw_1^{-n1} + \alpha_1^{-1})^{-1} + \alpha_2] \text{ pour } Sw \geq Sm \qquad (13)$$

$$Ct = C_1 [Sw_1^{-n1} + Sw_2^{-n2} \alpha_1^{-1})^{-1} + \alpha_2] \text{ pour } Sc \leq Sw \leq Sm \qquad (14).$$

L'indice de résistivité peut être calculé à partir des équations 12 à 14. Pour les valeurs mesurées de $f_1$, $f_2$ et $f_3$, il convient d'ajuster $n_1$, $n_2$, $\alpha_1$ et $\alpha_2$ aux données expérimentales. Tout comme dans le modèle DPC, on s'attend à trouver $\alpha_1$ de l'ordre de $f_2/f_1$ et $\alpha_2$ de l'ordre de $f_3/f_1$.

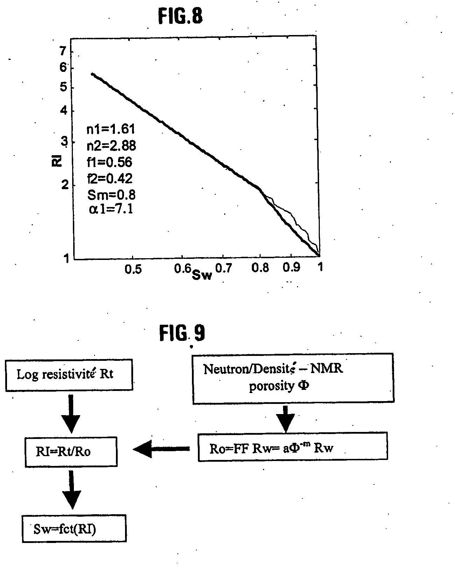

Ce modèle a été testé avec des courbes d'indice de résistivité mesurées sur des échantillons EL et BL. Sur l'échantillon EL (figure 7), le redressement de la courbe à saturation élevée est reproduit qualitativement à saturation élevée et l'aplatissement à faible saturation est bien reproduit. On observe une discontinuité à Sm (déduite du coude de la courbe de pression capillaire obtenue par injection de mercure) en raison d'une modification soudaine de la relation $Sw_1(Sw)$ (telle que celle montrée sur la figure 5). Après ajustage, les deux populations de pores dominantes présentent des caractéristiques similaires ($n_1=n_2=1,5$). La troisième population, qui se comporte comme un circuit parallèle, n'est pas envahie par l'huile et elle est seulement caractérisée par le rapport de conductivité $\alpha_2$. Sur l'échantillon BL (figure 8), la courbe RI peut être expliquée par un mécanisme similaire de résistance en série, mais avec une valeur Sm très élevée. Nous observons enfin que les deux réseaux possèdent des valeurs n très différentes et que le réseau 2 est beaucoup moins conducteur que le réseau 1 ($\alpha_1=7,1$).

## Revendications

1. Méthode pour déterminer les variations de l'indice de résistivité (RI) d'une famille d'échantillons de roche à structure poreuse complexe en fonction de la saturation en eau (Sw), en présence d'un fluide non conducteur, comportant les étapes suivantes :

   - pour chaque échantillon de la famille comprenant au moins un premier et un deuxième réseau de pores, on détermine la fraction volumique ($f_1$, $f_2$) occupée par chaque réseau de pores, par application aux différents échantillons d'une technique de relaxométrie de type RMN ;
   - pour chaque échantillon de la famille, on mesure par injection de mercure la distribution des seuils de pores donnant accès aux différents réseaux de pores pour déduire les valeurs des saturations moyennes (Sc, Sm);
   - on détermine expérimentalement sur un échantillon au moins de la famille servant de référence, les valeurs de coefficients ($n_1$, $n_2$) reliant la variation de sa résistivité électrique en fonction de sa saturation en eau (Sw) ; et
   - on détermine l'indice de résistivité (RI) de tous les échantillons de la famille en se basant sur la variation de paramètres décrivant l'agencement du réseau poreux ($f_1$, $f_2$, Sc, Sm) et en utilisant les valeurs des dits coefficients mesurés sur l'échantillon de référence.

2. Méthode selon la revendication 1, **caractérisée en ce que** l'on détermine à partir de l'échantillon de référence, les valeurs des coefficients ($n_1$, $n_2$) reliant la conductivité totale (Ct) de l'échantillon aux conductivité ($C_1$, $C_2$) des premier et deuxième réseaux de pores et aux saturations respectives en eau ($SW_1$, $SW_2$) des deux réseaux, l'indice de résistivité étant calculé à partir des fractions volumiques respectives ($f_1$, $f_2$) des deux réseaux de pores et de la valeur de la saturation moyenne (Sc) à partir de laquelle le réseau ayant les pores les plus petits, est envahi par le fluide non conducteur.

3. Méthode selon la revendication 1, **caractérisée en ce que** pour un échantillon comprenant un troisième réseau de pores, on détermine à partir de l'échantillon de référence, les valeurs des coefficients ($n_1$, $n_2$) reliant la conductivité totale (Ct) de l'échantillon aux conductivité ($C_1$, $C_2$) des premier et deuxième réseaux de pores et aux saturations respectives en eau ($SW_1$, $SW_2$) des deux premiers réseaux, l'indice de résistivité étant calculé à partir des fractions volumiques respectives ($f_1$, $f_2$, $f_3$) des trois réseaux de pores, de la valeur de la saturation moyenne (Sc) à partir de laquelle le réseau qui a les plus petits pores parmi les deux premiers réseaux de pores, est envahi par le fluide non conducteur, et de la valeur (Sm) à partir de laquelle le réseau ayant les plus grands pores, parmi les deux premiers réseaux de pores, est envahi par le fluide non conducteur.

**Patentansprüche**

1. Verfahren zur Bestimmung der Veränderungen des Resistivitäts- bzw. (spezifischen) Widerstandsindexes (RI) einer Familie von Gesteinsproben komplexer poröser Struktur als Funktion der Sättigung (Sw) mit Wasser, in Anwesenheit eines nicht leitenden Fluids, die folgenden Stufen umfassend:

    a. für jede Probe der wenigstens ein erstes und ein zweites Porennetz umfassenden Familie bestimmt man den Volumenanteil bzw. die Volumenfraktion ($f_1$, $f_2$), der bzw. die von jedem Porennetz eingenommen wird, durch Anwendung einer Relaxometrietechnik vom Typ RMN auf die verschiedenen Proben;
    b. für jede Probe der Familie misst man durch Einspritzen von Quecksilber die Verteilung der Schwellenwertporen, die Zugang zu den verschiedenen Porennetzen gewähren, um die mittleren Sättigungswerte (Sc, Sm) hieraus abzuleiten;
    c. man bestimmt experimentell an einer Probe wenigstens der als Bezug dienenden Familie die Werte der Koeffizienten ($n_1$, $n_2$), die die Veränderung ihres elektrischen (spezifischen) Widerstandes als Funktion ihrer Sättigung mit Wasser (Sw) verknüpfen; und
    d. man bestimmt den Resistivitätsindex (RI) sämtlicher Proben der Familie, indem man als Basis die Veränderung der Parameter nimmt, welche die Ausbildung des Porennetzes ($f_1$, $f_2$, Sc, Sm) beschreibt, und indem man die Werte der an der Bezugsprobe gemessenen Koeffizienten benutzt.

2. Verfahren nach Anspruch 1, **dadurch gekennzeichnet, dass** man, ausgehend von der Bezugsprobe, die Werte der Koeffizienten ($n_1$, $n_2$) bestimmt, welche die (spezifische) Gesamtleitfähigkeit (Ct) der Probe mit der (spezifischen) Leitfähigkeit ($C_1$, $C_2$) der ersten und zweiten Porennetze und mit den jeweiligen Sättigungen mit Wasser ($SW_1$, $SW_2$) der beiden Netze verknüpfen, wobei der Resistivitätsindex berechnet wird ausgehend von jeweiligen Volu-menanteilen ($f_1$, $f_2$) der beiden Porennetze und des mittleren Sättigungswertes (Sc), ab dem das nicht leitende Fluid in das Netz mit den kleinsten Poren eindringt.

3. Verfahren nach Anspruch 1, **dadurch gekennzeichnet, dass** für eine Probe mit einem dritten Porennetz man ausgehend von der Bezugsprobe die Koeffizientenwerte ($n_1$, $n_2$) bestimmt, welche die (spezifische) Gesamtleitfähigkeit (Ct) der Probe mit den Leitfähigkeiten ($C_1$, $C_2$) der ersten und zweiten Porennetze und mit den jeweiligen Sättigungen mit Wasser ($SW_1$, $SW_2$) der beiden ersten Netze verknüpfen, wobei der Resistivitätsindex berechnet wird ausgehend von den jeweiligen Volumenanteilen ($f_1$, $f_2$, $f_3$) der drei Porennetze, des Wertes der mittleren Sättigung (Sc) von dem aus das Netz, das die kleinsten Poren unter den beiden ersten Porennetzen hat, vom nicht leitenden Fluid durchdrungen wird, sowie ausgehend vom Wert (Sm), ab dem das über die größten Poren verfügende Netz, innerhalb der beiden ersten Porennetze, vom nicht leitenden Fluid durchdrungen wird.

**Claims**

1. Method for determining variations in the resistivity index (RI) of a family of rock samples of complex porous structure as a function of saturation with water (Sw), in the presence of a non-conductive fluid, including the following steps:

    - for each sample of the family comprising at least a first and a second pore network, the fraction by volume ($f_1$, $f_2$) is determined which is occupied by each pore network, by application to the different samples of a relaxometry technique of NMR type;
    - for each sample of the family, the distribution of the pore throats giving access to the different pore networks is measured by injection of mercury, to deduce the values of the mean saturations (Sc, Sm);
    - for at least one sample of the family acting as a reference, the values are determined experimentally of coefficients ($n_1$, $n_2$) linking the variation in its electrical resistivity as a function of its saturation with water (Sw); and
    - the resistivity index (RI) of all the samples of the family is determined based on the variation in parameters describing the layout of the pore network and using the values of the said coefficients measured for the reference sample.

2. Method as described in claim 1, **characterised by** the fact that the values are determined from the reference sample of the coefficients ($n_1$, $n_2$) linking the total conductivity (Ct) of the sample to the conductivity ($C_1$, $C_2$) of the first and second pore networks and to the respective saturations with water ($SW_1$, $SW_2$) of the two networks, the resistivity index being calculated from the respective fractions by volume ($f_1$, $f_2$) of the two pore networks and from the value of the mean saturation (Sc) from which the network having the smallest pores is invaded by the non-conductive fluid.

3. Method as described in claim 1, **characterised by** the fact that for a sample including a third pore network, the values are determined from the reference sample of the coefficients ($n_1$, $n_2$) linking the total conductivity (Ct) of the sample to the conductivity ($C_1$, $C_2$) of the first and second pore networks and to the respective saturations with water ($SW_1$, $SW_2$) of the first two networks, the resistivity index being calculated from the respective fractions by volume ($f_1$, $f_2$, $f_3$) of the three pore networks, from the value of the mean saturation (Sc) from which the network which has the smallest pores of the first two pore networks is invaded by the non-conductive fluid, and from the value (Sm) from which the network having the largest pores of the first two pore networks is invaded by the non-conductive fluid.

## FIG.1

## FIG.2

## FIG.3A

## FIG.3B

## FIG.4A

## FIG.4B

## FIG.5

## FIG.6

## FIG.7

## FIG.8

n1=1.61
n2=2.88
f1=0.56
f2=0.42
Sm=0.8
α1=7.1

## FIG.9

Log resistivité Rt

Neutron/Densité – NMR
porosity $\Phi$

$RI = Rt/Ro$

$Ro = FF\ Rw = a\Phi^{-m}\ Rw$

$Sw = fct(RI)$